# EUROPEAN PATENT SPECIFICATION

(11) **EP 0 475 482 B1**
(45) Date of publication and mention of the grant of the patent: **05.10.1994**
(21) Application number: 91202070.8
(22) Date of filing: 13.08.1991
(51) Int. Cl.: A61K 47/02, A61K 9/16, A61K 9/20, A61K 31/33

(54) **Stabilized solid chemical compositions**
Stabilisierte feste chemische Zusammensetzungen
Compositions chimiques solides stabilisées

(30) Priority: 13.09.1990 US 581859
(43) Date of publication of application: 18.03.1992
(73) Proprietor: Akzo Nobel N.V., 6824 BM Arnhem (NL)
(72) Inventor: de Haan, Pieter, NL-5343 XB Oss (NL); van der Ven, Cornelus Josephus, NL-5404 PH Uden (NL)

(56) References cited:
- EP-A- 0 380 021
- EP-B- 0 069 097

## Description

### Background of the Invention

Field. This invention relates to chemical compositions generally, especially to stable solid pharmaceutical preparations containing the water soluble acid addition salt of a poorly soluble basic compound.

State of the Art: Methods for making tablets and other solid or dry pharmaceutical preparations are well-known. For example in Chase, et al, Remington's Pharmaceutical Sciences, pp. 1553 - 1576 (16th ed. 1980, Mack Publ. Co. of Easton, PA, U.S.A.), methods of making tablets, capsules and pills and their respective components are described.

Two methods of making tablets are the "wet-granulation" and "dry-granulation" methods. The dry granulation method is especially suitable for medicinal compounds which are sensitive to moisture or are unable to withstand elevated drying temperatures associated with the wet-granulation methods.

Even with the use of these granulation methods however, the tableting of certain compounds (e.g. the water soluble acid addition salts of poorly soluble basic drugs) is less than ideal. Granulation processes using these compounds are not very "rugged", i.e. the processes have relatively strict tolerances making the granulation process extremely sensitive to changes in process variables (e.g. temperature and moisture changes). A process which is not very rugged has relatively strict operating tolerances which make the process sensitive to changes in processing variables. This is especially disadvantageous with regard to high temperature granulation processes which involve high temperatures and high humidities.

Even after tablets or other dry dosage pharmaceutical preparations are made containing the water soluble acid addition salts of poorly soluble basic drugs, the resulting preparations are generally not very stable. They discolour and/or degrade under certain conditions. For example, they may discolour or degrade upon exposure to light, relatively high humidity, or elevated temperatures. Color changes and degradation are tokens of instability. These tokens of instability may occur rather rapidly, sometimes within months, forcing a pharmacist or wholesaler storing the tablets to restock the product frequently.

The compound sodium pyrophosphate (Na₄P₂O₇) has been described as stabilizing certain calcium phosphate compounds. For example in U.S. Patent No. 2,287,699 to Moss et al, alkali metal pyrophosphates (e.g. Na₄P₂O₇) are used to prepare a stabilized form of dicalcium phosphate (e.g. CaHPO₄·2H₂O). In U.S. Patent No. 3,012,852 to Nelson et al, a process for producing "internally stabilized" dicalcium phosphate dihydrate using pyrophosphate ions is also disclosed.

Certain magnesium compounds are also known to stabilize calcium phosphate compounds. For example in U.S. Patent No. 2,018,410 to McDonald et al, trimagnesium phosphate, magnesium sulfate, magnesium stearate, and dimagnesium phosphate are all described as stabilizing hydrated dicalcium phosphate compounds substantially free from monocalcium phosphate for use in dentifrice preparations. In GB 1,548,465 to Hoechst Aktiengesellschaft, dimagnesium phosphate trihydrate is used to stabilize dicalcium phosphate dihydrate. In U.S. Patent 3,411,873 to Harnisch et al, a process for stabilizing dicalcium phosphate dihydrate by means of a magnesium phosphate is disclosed.

In German patent application DE 2,741,513 to J. H. Benckiser GmbH, a procedure is described for stabilizing calcium hydrogen phosphate dihydrate against hydrolysis using a diphosphonic acid or its water-soluble salt.

In European Patent 054,333 to Stauffer Chemical Co., fine particles of a calcium phosphate (e.g. calcium pyrophosphate) are compacted under pressure to form a sheet. The sheet may then be comminuted to give a granular material. This granular material may then be used as an excipient in pharmaceutical tablets or wafers.

US Patent No. 4,743,450 to Harris et al discloses pharmaceutical compositions containing a drug (i.e. an ACE inhibitor), an alkaline stabilizer, and a saccharide. Harris et al prefers water insoluble stabilizers such as magnesium carbonate, calcium carbonate, and magnesium silicate, which have not always proved adequate in trying to stabilize a high temperature, high humidity granulation process. Harris et al also specifically requires a saccharide component in the described compositions, which unnecessarily adds to the costs of such compositions. Harris et al furthermore does not disclose any methods of increasing the ruggedness of a granulation procedure, nor does the reference disclose rugged granulation procedures utilizing temperatures greater than 45°C. It is now found that when the drying temperature exceeds 45°C, such procedures become less rugged. The same criticality is found with regard to granulation temperatures.

European patent application 380,021 to Abbott Laboratories discloses that buffers may be used to obtain complete solubilization of certain drugs, and that these buffers can increase the thermal stability of a drug formulation during the drying step of the granulation process. It also discloses solid dosage forms having increased stability which may contain estropipate, a tromethamine buffer, and an additional alkaline buffering agent, such as dibasic sodium phosphate. The dosage forms may also contain an excipient, such as dibasic calcium phosphate.

To date no one has been able to economically increase the ruggedness of a high temperature mixing step of granulation procedures involving certain unstable chemical compounds (e.g. the water soluble acid addition salt of a poorly soluble basic compound).

### Summary of the Invention

Generally, the invention includes a dry chemical composition containing the water soluble acid addition salt of a poorly soluble basic compound (e.g. a drug); an excipient selected from microcrystalline cellulose, lactose, calcium hydrogen phosphate, and mixtures thereof; and a water soluble alkaline stabilizer. The dry composition is relatively more stable than a composition not containing the water soluble alkaline stabilizer. Including the water soluble alkaline stabilizer in the process for preparing the dry pharmaceutical preparation makes the process surprisingly more "rugged" with respect to stabilization of the drug, especially with regard to high temperature and high humidity granulation techniques.

Poorly soluble basic compounds for use in the invention include mianserin, apomorphine, chlorpromazine, imipramine, and promethazine. The particular acid addition salts of the chosen compounds will be at least partially capable of stabilization, in the selected excipient, by the particular stabilizer during storage and during a high temperature (>45° C) granulation process.

The preparation contains a sufficient amount of stabilizer (generally from 0.5 to 10% by weight of the dry pharmaceutical preparation) to stabilize the acid addition salt of the compound in the preparation for a desired time at a desired temperature. A typical stabilizer is soluble in water (> 2 milligrams/milliliter (mg/ml)); is alkaline in aqueous solution; and should be acceptable (e.g. pharmaceutically) for the intended use of the preparation.

The invention also includes a method of increasing the ruggedness of a granulation procedure utilizing temperatures greater than 45°C, involving a mixture of water soluble acid addition salts of poorly soluble basic compounds, and an excipient selected from microcrystalline cellulose, lactose, calcium hydrogen phosphate and mixtures thereof including: adding an acceptable alkaline compound having a water solubility of at least 2 milligrams / milliliter to the mixture, the acceptable alkaline compound being present in an amount from about 0.5 to 10 percent by the dry weight of the granulation mixture.

Besides increasing the ruggedness of the granulation procedure, the resulting compositions are surprisingly stable, having a longer shelf-life. Furthermore, the compounds contained within the resulting compositions do not migrate to the exterior of the composition as is sometimes the case with the prior art compositions, and fissures do not form in the compositions. Unexpectedly, pharmaceutical preparations made using the invention have better bioavailability.

### Description of the Preferred Embodiments

The stable dry composition is preferably a tablet, pill, capsule or powder. Tablets are the presently most preferred preparation, especially for pharmaceutical preparations.

The amount of drug in a dry pharmaceutical preparation will of course depend on the potency of the chosen drug and its intended use. The amount of drug used in a dosage unit is well-known to those skilled in the art, and depends on the particular acid addition salt used as the compound. Illustratively, tablets of mianserin hydrochloride contain from 10 - 60mg of the acid addition salt; tablets (hypodermic) of apomorphine hydrochloride contain from 5 to 6 milligrams; tablets of chlorpromazine hydrochloride contain from 10 to 200 milligrams; tablets of imipramine hydrochloride contain from 10 to 50 milligrams; and tablets of promethazine hydrochloride contain from 12.5 to 50 milligrams of promethazine hydrochloride.

Methods of making the described poorly soluble basic (e.g. amine) drug for use in the preparation are known. For example, methods of making imipramine are disclosed in U.S. Patent No. 2,554,736. Mianserin ("1,2,3,4,10,14b-hexahydro-2-methyl-dibenzo [c,f]pyrazino [1,2-a] azepine monohydrochloride), and similar compounds may be made according to the teachings of United States Patent Nos. 3,534,041 and 4,128,641. Other poorly soluble basic drugs which can form water soluble acid addition salts are readily commercially available.

Acid addition salts are derived from pharmaceutically acceptable acids, such as hydrochloric acid, sulfuric acid, phosphoric acid, acetic acid propionic acid, glycolic acid, maleic acid, fumaric acid, malonic acid, succinic acid, tartaric acid, lactic acid, citric acid, ascorbic acid, benzoic acid, methanesulfonic acid and the like. Acid addition salts may be obtained by reacting the poorly soluble basic drug with an appropriate acid in a suitable solvent.

The excipient is selected from microcrystalline cellulose, lactose, calcium hydrogen phosphate, and mixtures thereof. The amount of the selected excipient generally varies from about 30 to 80% by weight of the dry pharmaceutical preparation. Preferably the excipients will comprise 50 to 80 % by weight of the dry pharmaceutical preparation.

Compounds useful to stabilize the tablets once made, and to make the hereinafter described granulation process more rugged, include sodium bicarbonate (NaHCO₃), ammonium carbonate, anhydrous sodium carbonate (Na₂CO₃), sodium carbonate monohydrate, sodium tartrate, sodium potassium tartrate, sodium citrate (C₆H₅Na₃O₇·2H₂O), sodium hydroxide (NaOH), calcium acetate, sodium acetate, dibasic sodium phosphate (Na₂HPO₄·14H₂O), anhydrous dibasic sodium phosphate, diammonium hydrogen phosphate ((NH₄)₂HPO₄), calcium leavinulate (C₁₀H₁₄CaO₆·2H₂O), sodium pyrophosphate (Na₄P₂O₇), and mixtures thereof. Of these compounds sodium bicarbonate, ammonium carbonate, sodium citrate, dibasic sodium phosphate, anhydrous dibasic sodium biphosphate, diammonium hydrogen phosphate, sodium pyrophosphate and mixtures thereof are the most preferred.

The preparation preferably contains from 0.5 to 10% by weight of the dry pharmaceutical preparation of the stabilizer. Most preferably, the composition contains 1 to 5 % by weight of the pharmaceutical preparation (e.g. 1 to 5 % tablet weight).

As used herein, "stabilize" is a relative term. To stabilize means the ability to prevent or delay the onset of tokens of instability. For example, a composition would be deemed "stabilized" if, with the addition of a stabilizing compound ("stabilizer"), it took longer (e.g. 2 weeks instead of 1 week) to discolour in the presence of a destabilizing stimulus (e.g. storage of the solution at an elevated (60° centigrade (C)) temperature).

Methods for making dry pharmaceutical formulations are well-known. Methods for making powders are described in Remington's Pharmaceutical Sciences, pp. 1534 - 1552. Methods for making tablets, capsules and pills are disclosed in the same reference at pages 1553 -1584.

The preparations are preferably made in a wet-granulation process as described in Remington's Pharmaceutical Sciences at pages 1560 - 1563. The formulations can be stabilized for high temperature drying by first dry mixing the stabilizer with the tablet excipients before adding a granulation liquid (e.g. EXAMPLE I). Alternatively, the stabilizer may also be dissolved in the granulation liquid. Combinations of these two methods may also be used.

Although eminently suited for use in pharmaceutical dosage units, the invention has broader general applications not necessarily involved with the medical field. The invention can be used wherever high temperature, high humidity granulation techniques are required for compositions containing water soluble acid addition salts of poorly soluble chemical compounds (e.g. fertilizers, antiseptic or disinfectant tablets, herbicides, etc.).

The invention is further explained by reference to the following illustrative examples:

### EXAMPLE I

### Preparation of stabilized tablets

In a 300 liter high shear mixer, four batches are prepared according to the following FORMULATION 1:

| FORMULATION | 1 (kg) | 2 (kg) |
|---|---|---|
| mianserin·HCl | 10 | 10 |
| potato starch (intragranular) | 7.5 | 7.5 |
| methylcellulose | 1 | 1 |
| colloidal SiO₂ (intragranular) | 1 | 1 |
| NaHCO₃ | 2.5 | - |
| calcium hydrogenphosphate dihydrate | 74.2 | 76.8 |
| potato starch (intergranular) | 2.5 | 2.5 |
| colloidal SiO₂ (intergranular) | 1 | 1 |
| magnesium stearate | 0.2 | 0.2 |

The mianserin hydrochloride, colloidal silicon dioxide, calcium hydrogenphosphate dihydrate, dried potato starch and sodium bicarbonate are mixed for 3 minutes. The mixture is then granulated with an aqueous solution of methylcellulose (approximately 15 liters).

The four batches of granulates are dried according to procedures Ia, Ib, Ic and Id respectively.
Ia. A fluid bed dryer with granule bed temperature of about 45-50° C to a moisture level of approximately 3.2 % and filled into closed containers.
Ib. A fluid bed dryer with granule bed temperatures of about 30-35° C to moisture levels of approximately 3.2 % and filled into closed containers.
Ic. In a drying cabinet with granule bed temperatures of about 35-40° C to moisture levels of approximately 3.2 % and filled into closed containers.
Id. In a drying cabinet with granule bed temperatures of about 45-50° C to moisture levels of approximately 3.2 % and filled into closed containers.

The granules of Ia to Id are screened through a 3.5 and a 0.71 mm sieve respectively. The resulting batches are admixed with potato starch, colloidal silicon dioxide and finally with magnesium stearate, from which tablets of 100mg are prepared on a rotary press.

### EXAMPLE II

### Preparation of tablets without added stabilizer.

With the same equipment and the same process as described in EXAMPLE I, four batches are also made of the preparation of FORMULATION 2 (i.e. the formulation sans stabilizing agent). The granulates are dried in a fluid bed drier and in the drying cabinet in the same conditions as described in Ia to Id of EXAMPLE I, and are referred to as IIa to IId respectively. After screening the granules, and admixing with potato starch, colloidal silicon dioxide and magnesium stearate, 100mg tablets (total weight) are prepared.

### EXAMPLE III

### Ruggedness tests.

The respective batches (i.e. batches Ia through Id and IIa through IId) are analyzed immediately after tableting. Batches IIa and IId almost immediately yellowed, while all other batches remain a bright white.

### EXAMPLE IV

### Accelerated destabilization tests.

Various batches (i.e. batches Ia - Id and IIb and IIc) were analyzed by accelerated destabilization tests wherein the tablets were stored in closed containers and in unit dose strips at elevated temperatures for relatively short periods of time.
A. 40° C from 0 to 14 days - All batches maintained their bright white appearance for the period of time tested.
B. 50° C from 0 to 14 days - Batches Ia through Id maintained their bright white appearance for the period of time tested. Batches IIb and IIc began yellowing after 7 days in a closed container.
C. 60° C from 0 to 14 days - Batches Ia through Id maintained their bright white appearance for the period of time tested. Batches IIb and IIc began yellowing after 4 days in a closed container. Batches IIb and IIc began yellowing after 7 days in a unit dose strip.

### EXAMPLES V

### Preparation of stabilized tablets

In a 300 liter high shear mixer, four batches are prepared according to the following FORMULATION 3:

| FORMULATION | 3 (kg) | 4 (kg) |
|---|---|---|
| mianserin·HCl | 10 | 10 |
| potato starch (intragranular) | 7.5 | 7.5 |
| potato starch (intergranular) | 2.5 | 2.5 |
| methylcellulose | 1 | 1 |
| Na₂HPO₄ | - | 3 |
| colloidal silicon dioxide | 2 | 2 |
| magnesium stearate | 0.6 | 0.6 |
| Dibasic calcium phosphate dihydrate | qsad 100.0 | 100.0 |

In a high shear mixer (300 liters) Formulation 3 is prepared by mixing for four minutes the mianserin·HCl, half of the colloidal silicon dioxide, dibasic calcium phosphate dihydrate, and potato starch (6.5 kg (intragranular)). Then the granulation liquid, a mucilage of 1 kg of potato starch (intragranular) and 1 kg of methylcellulose in approximately 15 kg of heated water (90° C) is added and granulation is commenced. Formulation 3 is dried in a fluid bed drier with a granule bed temperature of about 30 to 35° C until a residual moisture content of about 3.2 % is attained, and are then filled into closed (air-tight) containers.

Exactly the same procedure for Formulation 4 is used with the exception that the 3 kg of Na₂HPO₄ is dry mixed with the other tablet constituents before addition of the granulation liquid.

The resulting batches are admixed with potato starch (2.5 kg), colloidal silicon dioxide, and finally with magnesium stearate. The respective batches are analyzed immediately after tableting. The batches prepared as FORMULATION 3 almost immediately yellowed, while the batches prepared as FORMULATION 4 remain a bright white.

### EXAMPLE VI

### Ruggedness test combined with accelerated destabilization test (worst case scenario).

Three batches were prepared on a 2 kilogram scale in a 10 liter Gral high shear mixer with the following formulations respectively:

| FORMULATION | 3 (kg) | 5&6 (kg) |
|---|---|---|
| mianserin·HCl | 10 | 10 |
| potato starch (intragranular) | 7.5 | 7.5 |
| potato starch (intergranular) | 2.5 | 2.5 |
| methylcellulose | 1 | 1 |
| Na₄P₂O₇ | - | 3 |
| colloidal silicon dioxide | 2 | 2 |
| magnesium stearate | 0.6 | 0.6 |
| Dibasic calcium phosphate dihydrate | qsad 100.0 | 100.0 |

Formulations 3, 5 and 6 were processed to granulates and tablets according to the with the hot mucilage of starch and methyl cellulose (90° C) as described in EXAMPLE V, with the exception that the granules have dried in a vacuum cabinet at approximately 35° C. The differences are:
Formulation 3: no stabilizer
Formulation 5: with stabilizer dissolved in the hot mucilage before granulation.
Formulation 6: with stabilizer dry mixed with the drug and the excipients before granulation with the hot mucilage.

### Results-

Tablets of Formulation 3, 5, and 6 were stored at 60° C for approximately 10 days. Tablets of Formulation 3 were discoloured, but the tables of Formulations 5 and 6 remain a bright white, irrespective of the severe stress during hot granulation and storage of the tablets in extreme conditions.

### EXAMPLE VII

### Bioavailability tests

The tablets resulting from Formulations 2, 5 and 6 were subject to a standard test for predicting bioavailability (Dissolution in 0.1N HCl with a USP paddle, 50 rpm). Formulations 5 and 6 dissolved faster than Formulation 2, showing the advantage of using "hot" granulation steps.

Reference herein to specific embodiments or examples should not be interpreted as limitations to the scope of the invention, which is defined by the appended claims.

## Claims (Claims for the following Contracting State(s): AT, BE, CH, LI, DE, DK, FR, GB, IT, NL, SE)

1. A dry chemical composition which has been granulated comprising: a water soluble acid addition salt of a poorly soluble basic compound; an excipient selected from microcrystalline cellulose, lactose, and calcium hydrogen phosphate in an amount from about 30 to 80 percent by weight of the dry chemical composition; and a water soluble alkaline stabilizer, used to stabilize the composition during granulation, in an amount from about 0.5 to 10 percent by weight of the dry chemical composition.

2. The dry chemical composition of claim 1 wherein said poorly soluble basic compound is selected from apomorphine, chlorpromazine, imipramine, promethazine, and mianserin.

3. The dry chemical composition of claim 2 wherein said poorly soluble basic compound is mianserin.

4. The dry chemical composition of claim 3 containing from about 1 to 5 per cent by weight of the dry chemical composition a water soluble alkaline stabilizer.

5. The dry chemical composition of claim 1 wherein said stabilizer is selected from sodium bicarbonate, ammonium carbonate, sodium citrate, dibasic sodium phosphate, anhydrous dibasic sodium biphosphate, diammonium hydrogen phosphate, sodium pyrophosphate and mixtures thereof.

6. A method of increasing the ruggedness of a granulation procedure utilizing temperatures greater than 45°C and involving a mixture of a water soluble acid addition salt of poorly soluble basic compound, and an excipient selected from microcrystalline cellulose, lactose, calcium hydrogen phosphate and mixtures thereof comprising: adding an alkaline compound having a water solubility of at least 2 milligrams / milliliter to the mixture before granulation, said alkaline compound being present in an amount from about 0.5 to 10 percent by the dry weight of the granulation mixture.

7. The method of claim 6 wherein said alkaline compound has a solubility in water of at least 4 milligrams / milliliter.

8. The method of claim 7 wherein said alkaline compound is present in an amount from about 1 to 5 per cent by weight of the granulation mixture.

9. The use of an alkaline compound having a water solubility of at least 2 milligrams / milliliter as a stabilizer in a high temperature granulation process involving a composition containing a mixture of a water soluble acid addition salt of poorly soluble basic compound, and an excipient selected from microcrystalline cellulose, lactose, calcium hydrogen phosphate and mixtures thereof.

10. A stabilized dry pharmaceutical preparation comprising:
the water soluble acid addition salt of an poorly soluble basic drug selected from apomorphine, chlorpromazine, imipramine, and promethazine;
from about 30 to 80 per cent by weight of the dry pharmaceutical preparation, an excipient selected from the group consisting of microcrystalline cellulose, lactose, and calcium hydrogen phosphate; and
from about 0.5 to 10 per cent by weight of the dry pharmaceutical preparation a water soluble alkaline stabilizer selected from sodium bicarbonate, ammonium carbonate, sodium citrate, dibasic sodium phosphate, anhydrous dibasic sodium phosphate, diammonium hydrogen phosphate, sodium pyrophosphate and mixtures thereof, said stabilizer used to stabilize said preparation during a granulation procedure.

## Claims (Claims for the following Contracting State(s): ES, GR)

1. Process for the preparation of a dry granulated chemical composition comprising:
mixing a water soluble acid addition salt of a poorly soluble basic compound and
an excipient selected from microcrystalline cellulose, lactose, and calcium hydrogen phosphate in an amount from about 30 to 80 percent by weight of the dry chemical composition and
a water soluble alkaline stabilizer, used to stabilize the composition during granulation, in an amount from about 0.5 to 10 percent by weight of the dry chemical composition;
followed by granulation of this mixture.

2. Process of claim 1 wherein said poorly soluble basic compound is selected from apomorphine, chlorpromazine, imipramine, promethazine, and mianserin.

3. Process of claim 2 wherein said poorly soluble basic compound is mianserin.

4. Process of claim 3 containing a water soluble alkaline stabilizer in an amount from about 1 to 5 percent by weight of the dry chemical composition.

5. Process of claim 1 wherein said stabilizer is selected from sodium bicarbonate, ammonium carbonate, sodium citrate, dibasic sodium phosphate, anhydrous dibasic sodium biphosphate, diammonium hydrogen phosphate, sodium pyrophosphate and mixtures thereof.

6. A method of increasing the ruggedness of a granulation procedure utilizing temperatures greater than 45°C and involving a mixture of a water soluble acid addition salt of poorly soluble basic compound, and an excipient selected from microcrystalline cellulose, lactose, calcium hydrogen phosphate and mixtures thereof comprising: adding an alkaline compound having a water solubility of at least 2 milligrams / milliliter to the mixture before granulation, said alkaline compound being present in an amount from about 0.5 to 10 percent by the dry weight of the granulation mixture.

7. The method of claim 6 wherein said alkaline compound has a solubility in water of at least 4 milligrams / milliliter.

8. The method of claim 7 wherein said alkaline compound is present in an amount from about 1 to 5 per cent by weight of the granulation mixture.

9. The use of an alkaline compound having a water solubility of at least 2 milligrams / milliliter as a stabilizer in a high temperature granulation process involving a composition containing a mixture of a water soluble acid addition salt of poorly soluble basic compound, and an excipient selected from microcrystalline cellulose, lactose, calcium hydrogen phosphate and mixtures thereof.

10. Process for the preparation of a dry pharmaceutical preparation comprising:
mixing
the water soluble acid addition salt of an poorly soluble basic drug selected from apomorphine, chlorpromazine, imipramine, and promethazine;
from about 30 to 80 per cent by weight of the dry pharmaceutical preparation, an excipient selected from the group consisting of microcrystalline cellulose, lactose, and calcium hydrogen phosphate; and
from about 0.5 to 10 per cent by weight of the dry pharmaceutical preparation a water soluble alkaline stabilizer selected from sodium bicarbonate, ammonium carbonate, sodium citrate, dibasic sodium phosphate, anhydrous dibasic sodium phosphate, diammonium hydrogen phosphate, sodium pyrophosphate and mixtures thereof, said stabilizer used to stabilize said preparation during a granulation procedure.

## Patentansprüche (Patentansprüche für folgende(n) Vertragsstaat(en): AT, BE, CH, LI, DE, DK, FR, GB, IT, NL, SE)

1. Eine trockene chemische Zusammensetzung, die granuliert wurde, welche umfasst: ein wasserlösliches Säureadditionssalz einer schwerlöslichen basischen Verbindung; einen Arzneimittelträger ausgewählt aus mikrokristalliner Cellulose, Laktose und Calziumhydrogenphosphat in einer Menge von ungefähr 30 bis 80 Gewichtsprozent der trockenen chemischen Zusammensetzung; und einen wasserlöslichen alkalischen Stabilisator in einer Menge von ungefähr 0,5 bis 10 Gewichtsprozent der trockenen chemischen Zusammensetzung, der verwendet wird, um die Zusammensetzung während der Granulierung zu stabilisieren.

2. Die trockene chemische Zusammensetzung gemäss Anspruch 1, worin die schwerlösliche basische Verbindung ausgewählt wird aus Apomorphin, Clorpromazin, Imipramin, Promethazin und Mianserin.

3. Die trockene chemische Zusammensetzung gemäss Anspruch 2, worin besagte schwerlösliche basische Verbindung Mianserin ist.

4. Die trockene chemische Zusammensetzung gemäss Anspruch 3, die einen wasserlöslichen alkalischen Stabilisator zu ungefähr 1 bis 5 Gewichtsprozent der trockenen chemischen Zusammensetzung enthält.

5. Die trockene chemische Zusammensetzung gemäss Anspruch 1, worin besagter Stabilisator ausgewählt wird aus Natriumhydrogencarbonat, Ammoniumcarbonat, Natriumcitrat, Natriummonohydrogenorthophosphat, wasserfreies Natriummonohydrogenorthophosphat, Diammoniummonohydrogenorthophosphat, Natriumpyrophosphat und Mischungen davon.

6. Ein Verfahren zur Erhöhung der Widerstandsfähigkeit eines Granulierungsverfahrens, das Temperaturen höher als 45°C anwendet und eine Mischung eines wasserlöslichen Säureadditionssalzes einer schwerlöslichen basischen Verbindung und einen Arzneimittelträger ausgewählt aus mikrokristalliner Zellulose, Laktose, Calziumhydrogenphosphat und Mischungen davon einschliesst, welches umfasst: Zugabe einer alkalischen Verbindung, die eine Wasserlöslichkeit von mindestens 2 Milligramm/Milliliter der Mischung vor der Granulierung hat, wobei besagte alkalische Verbindung in einer Menge von ungefähr 0,5 bis 10 Prozent des Trockengewichtes der Granulationsmischung vorhanden ist.

7. Das Verfahren gemäss Anspruch 6, worin besagte alkalische Verbindung eine Löslichkeit in Wasser von mindestens 4 Milligramm/Milliliter hat.

8. Das Verfahren gemäss Anspruch 7, worin besagte alkalische Verbindung in einer Menge von ungefähr 1 bis 5 Gewichtsprozent der Granulierungsmischung vorhanden ist.

9. Die Verwendung einer alkalischen Verbindung, die eine Wasserlöslichkeit von mindestens 2 Milligramm/Milliliter hat, als Stabilisator in einem Hochtemperaturgranulierungsverfahren, das eine Verbindung einschliesst, die eine Mischung aus einem wasserlöslichen Säureadditonssalz einer schwerlöslichen basischen Verbindung und einem Arzneimittelträger, ausgewählt aus mikrokristalliner Cellulose, Laktose, Calziumhydrogenphosphat und Mischungen davon, enthält.

10. Ein stabilisiertes, trockenes, pharmazeutisches Präparat, welches umfasst:
das wasserlösliche Additionssalz eines schwerlöslichen basischen Arzneimittels ausgewählt aus Apomorphin, Chlorpromazin, Imipramin und Promethazin; einen Arzneimittelträger zu ungefähr 30 bis 80 Gewichtsprozent des trockenen, pharmazeutischen Präparats, ausgewählt aus der Gruppe bestehend aus mikrokristalliner Cellulose, Laktose und Calziumhydrogenphosphat; und einen wasserlöslichen alkalischen Stabilisator zu 0,5 bis 10 Gewichtsprozent des trockenen, pharmazeutischen Präparats, ausgewählt aus Natriumhydrogencarbonat, Ammoniumcarbonat, Natriumcitrat, Natriummonohydrogenorthophosphat, wasserfreies Natriummonohydrogenorthophosphat, Diammoniummonohydrogenorthophosphat, Natriumpyrophosphat und Mischungen davon, unter Anwendung besagten Stabilisators zum Stabilisieren des Präparats während eines Granulierungsverfahrens.

## Patentansprüche (Patentansprüche für folgende(n) Vertragsstaat(en): ES, GR)

1. Verfahren zur Herstellung einer trockenen, granulierten, chemischen Zusammensetzung, welches umfasst:
Mischen eines wasserlöslichen Säureadditionssalzes einer schwerlöslichen basischen Verbindung und eines Arzneimittelträgers in einer Menge von ungefähr 30 bis 80 Gewichtsprozent der trockenen, chemischen Zusammensetzung ausgewählt aus mikrokristalliner Cellulose, Laktose und Calziumhydrogenphosphat und eines wasserlöslichen alkalischen Stabilisators in einer Menge von 0,5 bis 10 Gewichtsprozent der trockenen chemischen Zusammensetzung, der verwendet wird um die Zusammensetzung während der Granulierung zu stabilisieren;
gefolgt von einer Granulierung dieser Mischung.

2. Verfahren gemäss Anspruch 1, worin besagte schwerlösliche basische Verbindung aus Apomorphin, Chlorpromazin, Imipramin, Promethazin und Mianserin ausgewählt wird.

3. Verfahren gemäss Anspruch 2, worin besagte schwerlösliche Verbindung Mianserin ist.

4. Verfahren gemäss Anspruch 3, das einen wasserlöslichen alkalischen Stabilisator in einer Menge von 1 bis 5 Gewichtsprozent der trockenen chemischen Zusammensetzung enthält.

5. Verfahren gemäss Anspruch 1, worin besagter Stabilisator aus Natriumhydrogencarbonat, Ammoniumcarbonat, Natriumcitrat, Natriummonohydrogenorthophosphat, wasserfreies Natriummonohydrogenorthophosphat, Diammoniummonohydrogenorthophosphat, Natriumpyrophosphat und Mischungen davon ausgewählt wird.

6. Ein Verfahren zur Erhöhung der Widerstandsfähigkeit eines Granulierungsverfahrens, das Temperaturen höher als 45°C verwendet und das eine Mischung eines wasserlöslichen Säureadditionssalzes einer schwerlöslichen basischen Verbindung und einen Arzneimittelträger ausgewählt aus mikrokristalliner Cellulose, Laktose, Calziumhydrogenphosphat und Mischungen davon einschliesst, welches umfasst: Zugabe einer alkalischen Verbindung, die eine Wasserlöslichkeit von mindestens 2 Milligramm/Milliliter der Mischung vor der Granulierung hat, wobei besagte alkalische Verbindung in einer Menge von 0,5 bis 10 Prozent des Trockengewichts der Granulierungsmischung vorhanden ist.

7. Das Verfahren gemäss Anspruch 6, worin besagte alkalische Verbindung eine Löslichkeit in Wasser von mindestens 4 Milligramm/Milliliter hat.

8. Das Verfahren gemäss Anspruch 7, worin besagte alkalische Verbindung in einer Menge von ungefähr 1 bis 5 Gewichtsprozent der Granulierungsmischung vorhanden ist.

9. Die Verwendung einer alkalischen Verbindung, die eine Wasserlöslichkeit von mindestens 2 Milligramm/Milliliter hat, als Stabilisator in einem Hochtemperaturgranulierungsprozess, der eine Zusammensetzung einschliesst, die eine Mischung eines wasserlöslichen Säureadditionssalzes einer schwerlöslichen basischen Verbindung und einen Arzneimittelträger, ausgewählt aus mikrokristalliner Cellulose, Laktose, Calziumhydrogenphosphat und Mischungen davon einschliesst.

10. Verfahren zur Herstellung eines trockenen pharmazeutischen Präparats, welches umfasst:
Mischen eines wasserlöslichen Säureadditionssalzes eines schwerlöslichen basischen Arzneimittels ausgewählt aus Apomorphin, Chlorpromazin, Imipramin und Promethazin; eines Arzneimittelträgers zu ungefähr 30 bis 80 Gewichtsprozent des trockenen, pharmazeutischen Präparats ausgewählt aus der Gruppe bestehend aus mikrokristalliner Cellulose, Laktose und Calziumhydrogenphosphat; und eines wasserlöslichen alkalischen Stabilisators zu 0,5 bis 10 Gewichtsprozent des trockenen, pharmazeutischen Präparats, ausgewählt aus Natriumhydrogencarbonat, Ammoniumcarbonat, Natriumcitrat, Natriummonohydrogenorthophosphat, wasserfreies Natriummonohydrogenorthophosphat, Diammoniummonohydrogenorthophosphat, Natriumpyrophosphat und Mischungen davon, unter Verwendung des Stabilisators zum Stabilisieren des Präparates während eines Granulierungsverfahrens.

## Revendications (Revendications pour l'(les) Etat(s) contractant(s) suivant(s): AT, BE, CH, LI, DE, DK, FR, GB, IT, NL, SE)

1. Une composition chimique sèche qui a été granulée comprenant: un sel d'addition d'acide hydrosoluble d'un composé basique faiblement soluble; un excipient sélectionné parmi cellulose microcristalline, lactose, hydrogénophosphate de calcium en une quantité d'environ 30 à 80% en poids par rapport au poids de la composition chimique sèche; et un agent stabilisant alcalin hydrosoluble, utilisé pour stabiliser la composition durant la granulation, en une quantité d'environ 0,5 à 10% en poids par rapport au poids de la composition chimique sèche.

2. La composition chimique sèche selon la revendication 1, dans laquelle ledit composé basique faiblement soluble est sélectionné parmi apomorphine, chlorpromazine, imipramine, prométhazine et miansérine.

3. La composition chimique sèche selon la revendication 2, caractérisée en ce que ledit composé basique faiblement soluble est la miansérine.

4. La composition chimique sèche selon la revendication 3, contenant de 1 à 5% en poids d'un agent stabilisant alcalin hydrosoluble par rapport au poids de la composition chimique sèche.

5. La composition chimique sèche selon la revendication 1, dans laquelle ledit agent stabilisant est sélectionné parmi carbonate de sodium, carbonate d'ammonium, citrate de sodium, phosphate de sodium dibasique, biphosphate de sodium dibasique anhydre, hydrogénophosphate de diammonium, pyrophosphate de sodium et leurs mélanges.

6. Un procédé pour augmenter la "flexibilité" d'un procédé de granulation utilisant des températures supérieures à 45°C et mettant en oeuvre un mélange constitué d'un sel d'addition d'acide hydrosoluble d'un composé basique faiblement soluble, et d'un excipient sélectionné parmi cellulose microcristalline, lactose, hydrogénophosphate de calcium et leurs mélanges, comprenant: l'addition d'un composé alcalin présentant une solubilité dans l'eau d'au moins 2 mg/ml au mélange avant la granulation, ledit composé alcalin étant présent en une quantité d'environ 0,5 à 10% en poids par rapport au poids du mélange de granulation.

7. Le procédé selon la revendication 6, dans lequel ledit composé alcalin présente une solubilité dans l'eau d'au moins 4 mg/ml.

8. Le procédé selon la revendication 7, dans lequel ledit composé alcalin est présent en une quantité de 1 à 5% en poids par rapport au poids du mélange de granulation.

9. Utilisation d'un composé alcalin présentant une solubilité dans l'eau d'au moins 2 mg/ml en tant qu'agent stabilisant dans un procédé de granulation à haute température mettant en oeuvre une composition contenant un mélange d'un sel d'addition d'acide hydrosoluble d'un composé basique faiblement soluble, et d'un excipient sélectionné parmi cellulose microcristalline, lactose, hydrogénophosphate de calcium et leurs mélanges.

10. Une préparation pharmaceutique sèche stabilisée, comprenant:
- le sel d'addition d'acide hydrosoluble d'un médicament basique faiblement soluble sélectionné parmi apomorphine, chlorpromazine, imipramine et prométhazine;
- d'environ 30 à 80% en poids d'un excipient sélectionné dans le groupe consistant en cellulose microcristalline, lactose et hydrogénophosphate de calcium par rapport au poids de la préparation pharmaceutique sèche; et
- d'environ 0,5 à 10% en poids d'un agent stabilisant alcalin hydrosoluble sélectionné parmi bicarbonate de sodium, carbonate d'ammonium, citrate de sodium, phosphate de sodium dibasique. phosphate de sodium dibasique anhydre, hydrogénophosphate de diammonium, pyrophosphate de sodium et leurs mélanges, par rapport au poids de la composition pharmaceutique sèche; ledit agent stabilisant étant utilisé pour stabiliser ladite préparation durant le procédé de granulation.

## Revendications (Revendications pour l'(les) Etat(s) contractant(s) suivant(s): ES, GR)

1. Procédé de préparation d'une composition chimique sèche granulée sèche comprenant: le mélange d'un sel d'addition d'acide hydrosoluble d'un composé basique faiblement soluble et d'un excipient sélectionné parmi cellulose microcristalline, lactose, hydrogénophosphate de calcium, en une quantité d'environ 30 à 80% en poids par rapport au poids de la composition chimique sèche; et un agent stabilisant alcalin hydrosoluble, utilisé pour stabiliser la composition durant la granulation, en une quantité d'environ 0,5 à 10% en poids par rapport au poids de la composition chimique sèche; suivi de la granulation de ce mélange.

2. Procédé selon la revendication 1, dans lequel ledit composé basique faiblement soluble est sélectionné parmi apomorphine, chlorpromazine, imipramine, prométhazine et miansérine.

3. Procédé selon la revendication 2, dans lequel ledit composé basique faiblement soluble est la miansérine.

4. Procédé selon la revendication 3, contenant de 1 à 5% en poids d'un agent stabilisant alcalin hydrosoluble par rapport au poids de la composition chimique sèche.

5. Procédé selon la revendication 1, dans lequel ledit agent stabilisant est sélectionné parmi carbonate de sodium, carbonate d'ammonium, citrate de sodium, phosphate de sodium dibasique, biphosphate de sodium dibasique anhydre, hydrogénophosphate de diammonium, pyrophosphate de sodium et leurs mélanges.

6. Un procédé pour augmenter la "flexibilité" d'un procédé de granulation utilisant des températures supérieures à 45°C et mettant en oeuvre un mélange constitué d'un sel d'addition d'acide hydrosoluble d'un composé basique faiblement soluble, et d'un excipient sélectionné parmi cellulose microcristalline, lactose, hydrogénophosphate de calcium et leurs mélanges, comprenant: l'addition d'un composé alcalin présentant une solubilité dans l'eau d'au moins 2 mg/ml au mélange avant la granulation, ledit composé alcalin étant présent en une quantité d'environ 0,5 à 10% en poids par rapport au poids du mélange de granulation.

7. Le procédé selon la revendication 6, dans lequel ledit composé alcalin présente une solubilité dans l'eau d'au moins 4 mg/ml.

8. Le procédé selon la revendication 7, dans lequel ledit composé alcalin est présent en une quantité de 1 à 5% en poids par rapport au poids du mélange de granulation.

9. Utilisation d'un composé alcalin présentant une solubilité dans l'eau d'au moins 2 mg/ml en tant qu'agent stabilisant dans un procédé de granulation à haute température mettant en oeuvre une composition contenant un mélange d'un sel d'addition d'acide hydrosoluble d'un composé basique faiblement soluble, et d'un excipient sélectionné parmi cellulose microcristalline, lactose, hydrogénophosphate de calcium et leurs mélanges.

10. Un procédé de préparation d'une préparation pharmaceutique sèche, comprenant:
- le mélange du sel d'addition d'acide hydrosoluble d'un médicament basique faiblement soluble sélectionné parmi apomorphine, chlorpromazine, imipramine et prométhazine;
- d'environ 30 à 80% en poids d'un excipient sélectionné dans le groupe consistant en cellulose microcristalline, lactose et hydrogénophosphate de calcium par rapport au poids de la préparation pharmaceutique sèche; et
- d'environ 0,5 à 10% en poids d'un agent stabilisant alcalin hydrosoluble sélectionné parmi bicarbonate de sodium, carbonate d'ammonium, citrate de sodium, phosphate de sodium dibasique, phosphate de sodium dibasique anhydre, hydrogénophosphate de diammonium, pyrophosphate de sodium et leurs mélanges, par rapport au poids de la composition pharmaceutique sèche; ledit agent stabilisant étant utilisé pour stabiliser ladite préparation durant le procédé de granulation.
